# EUROPEAN PATENT APPLICATION

(11) **EP 3 725 766 A1**
(43) Date of publication of application: **21.10.2020**
(21) Application number: 18887421.8
(22) Date of filing: 16.10.2018
(51) Int. Cl.: C07C 213/10, C07C 213/08, C07C 219/06, C07B 57/00

(54) **METHOD FOR PRODUCING 2-OCTYLGLYCINE ESTER HAVING OPTICAL ACTIVITY**

(30) Priority: 12.12.2017 KR 20170170315
(71) Applicant: Aminologics Co., Ltd., Seoul 06244 (KR)
(72) Inventor: BYUN, Il Suk, Seongnam-si, Gyeonggi-do 13587 (KR); LEE, Jin Suk, Cheongju-si, Chungcheongbuk-do 28801 (KR); KIM, Won Sup, Sejong 30098 (KR)
(74) Representative: Cabinet Becker et Associés
(86) International application number: PCT/KR2018/012179
(87) International publication number: WO 2019/117446

(57) **Abstract**

The present invention relates to a method for producing a 2-octylglycine ester having optical activity from a racemic 2-octyl-DL-glycine ester and, more particularly, to a method for producing a 2-octyl-L-glycine ester or a 2-octyl-D-glycine ester by using a chiral mandelic acid as an optical resolution agent.

## Description

### [TECHNICAL FIELD]

The present invention relates to a method for producing a 2-octylglycine ester having optical activity from a racemic 2-octyl-DL-glycine ester, and more specifically, a method for producing a 2-octyl-L-glycine ester or 2-octyl-D-glycine ester using a chiral mandelic acid as a chiral resolving agent.

### [BACKGROUND ART]

The 2-octylglycine ester having optical activity is represented by Formula 1 below and is a chiral compound having a chiral center. 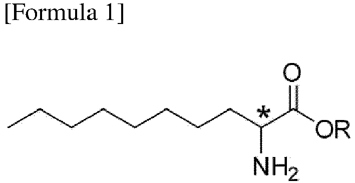
wherein * is a chiral center, and
R is an alkyl group having 1 to 4 carbon atoms.

2-octylglycine having optical activity is a key raw material for production of a peptide drug used as an anticancer or antibiotic drug (Tetrahedron, 66, 3427 (2010); J. Org. Chem, 72, 5146 (2007); Bioconjugate Chemistry, 25, 750 (2014)).

Conventionally known techniques to produce 2-octylglycine having optical activity can be broadly categorized into two types. One is a method employing enzymatic biological optical resolution reaction, and the other one is a method using asymmetric chemical reaction.

Methods for producing 2-octylglycine having optical activity using enzymatic biological reaction are known. For example, a method is known for producing it through hydrolysis reaction using an enzyme derived from *Aspergillus* from N-chloroacetyl-2-octylglycine or N-acetyl-2-octylglycine as a raw material (Agricultural and Biological Chemistry, 46, 2319 (1982); J. Med. Chem., 32, 289 (1989)), but it poses a difficulty that an enzyme that is difficult to handle in biological processes has to be used.

Additionally, a production method using a chiral pyridoxamine auxiliary (Organic Letters, 17, 5784 (2015); J. Am. Chem. Soc., 138, 10730 (2016)) and a production method using a chiral dihydropyrazine auxiliary (Organic Letters, 5, 5047 (2003); Tetrahedron, 66, 3427 (2010)) were reported as production methods involving asymmetric chemical reaction using a chiral auxiliary, but they have a disadvantage that it is difficult to synthesize the chiral auxiliary to be used.

### [PRIOR ART DOCUMENTS]

### [NON-PATENT DOCUMENTS]

(NON-PATENT DOCUMENT 1) Tetrahedron, 66, 3427 (2010)
(NON-PATENT DOCUMENT 2) J. Org. Chem, 72, 5146 (2007)
(NON-PATENT DOCUMENT 3) Bioconjugate Chemistry, 25, 750 (2014)
(NON-PATENT DOCUMENT 4) Agricultural and Biological Chemistry, 46, 2319 (1982)
(NON-PATENT DOCUMENT 5) J. Med. Chem., 32, 289 (1989)
(NON-PATENT DOCUMENT 6) Organic Letters, 17, 5784 (2015)
(NON-PATENT DOCUMENT 7) J. Am. Chem. Soc., 138, 10730 (2016)
(NON-PATENT DOCUMENT 8) Organic Letters, 5, 5047 (2003)

### [DETAILED DESCRIPTION OF THE INVENTION]

### [PROBLEM TO BE SOLVED]

In order to solve the above-mentioned problems,

The present invention has an object of providing a method for producing a 2-octylglycine ester having optical activity from a racemic 2-octyl-DL-glycine ester using a chiral mandelic acid as a chiral resolving agent.

### [MEANS FOR SOLVING THE PROBLEM]

In order to achieve the object,

The present invention provides a method for producing a 2-octylglycine ester having optical activity, the 2-octylglycine ester being represented by Formula 1, wherein a racemic 2-octyl-DL-glycine ester is reacted with a chiral mandelic acid represented by Formula 2, which is a chiral resolving agent: 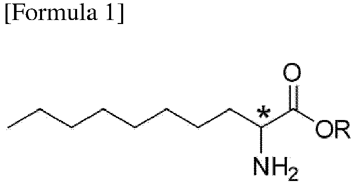
wherein R is an alkyl group having 1 to 4 carbon atoms, and
* is a chiral center;

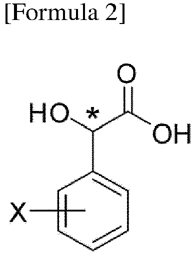

wherein X is hydrogen; a methoxy group; an alkyl group having 1 to 3 carbon atoms; fluorine; chlorine; bromine; or a cyano group, and
* is a chiral center.

### [EFFECT OF THE INVENTION]

The method for producing a 2-octylglycine ester having optical activity according to the present invention can produce a 2-octylglycine ester having optical activity with high optical purity.

Additionally, a chiral mandelic acid, which is a chiral resolving agent, used in the method for producing a 2-octylglycine ester having optical activity according to the present invention has high commercial availability and thus is suitable for mass production of a 2-octylglycine ester having optical activity.

Also, the method for producing a 2-octylglycine ester having optical activity according to the present invention is a very easy method, which results in advantages that it can be readily applicable and allows mass production.

### [BRIEF DESCRIPTION OF THE DRAWINGS]

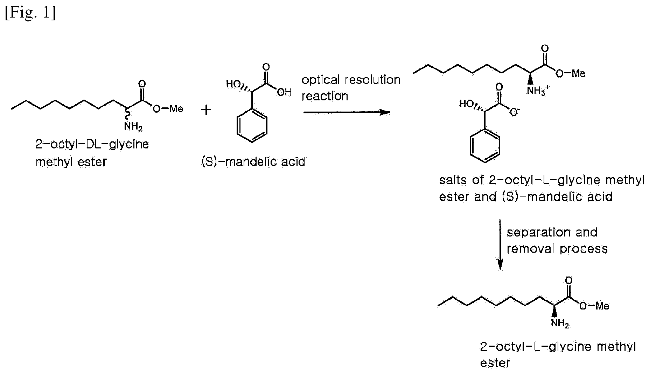

Fig. 1 is a schematic view showing a representative method for producing a 2-octyl-L-glycine methyl ester through optical resolution reaction and a hydrolysis process using a (S)-mandelic acid as a chiral resolving agent.

### [DETAILED DESCRITPION TO CARRY OUT THE INVENTION]

Hereinafter, the present invention is described in further detail.

In general, a chemical optical resolution reaction using a commercially readily available chiral organic acid is easy to process compared to a biological process due to the nature of the chemical process, and has an advantage that a chiral auxiliary, which is difficult to synthesize, is unnecessary; so it is suitable for mass production. However, it is very important and difficult to find and select a suitable chiral organic acid to be used as a chiral resolving agent.

In this light, the inventors of the present invention explored an optical resolution effect of a racemic 2-octylglycine ester using various chiral organic acids such as camphorsulfonic acid, dibenzoyl tartaric acid, camphoric acid, malic acid, pyroglutamic acid, and tartaric acid, which are chiral organic acids, as a chiral resolving agent, and found that surprisingly a chiral mandelic acid has a very good optical resolution effect to complete the present invention.

By applying the chiral mandelic acid used as a chiral resolving agent for optical resolution of a racemic 2-octyl-DL-glycine ester, a 2-octylglycine ester having optical activity was obtained with high optical purity.

That is, the present invention relates to a method for producing a 2-octylglycine ester having optical activity, the 2-octylglycine ester being represented by Formula 1, wherein a racemic 2-octyl-DL-glycine ester is reacted with a chiral mandelic acid represented by Formula 2, which is a chiral resolving agent: 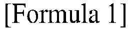 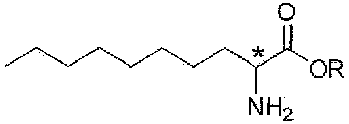
wherein R is an alkyl group having 1 to 4 carbon atoms, and
* is a chiral center;

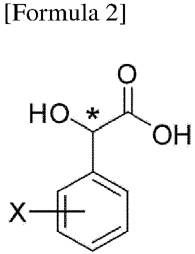

wherein X is hydrogen; a methoxy group; an alkyl group having 1 to 3 carbon atoms; fluorine; chlorine; bromine; or a cyano group, and
* is a chiral center.

Meanwhile, the 2-octylglycine ester having optical activity according to Formula 1 can be more specifically represented by a 2-octyl-L-glycine ester of Formula 3 and a 2-octyl-D-glycine ester of Formula 4: 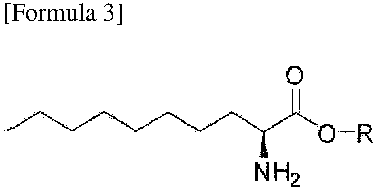 where R is an alkyl group having 1 to 4 carbon atoms; 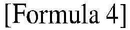 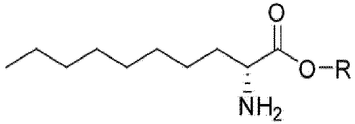 wherein R is an alkyl group having 1 to 4 carbon atoms.

More specifically, the present invention relates to a method for producing a 2-octyl-L-glycine ester or a 2-octyl-D-glycine ester as a 2-octylglycine ester having optical activity by optical resolution of a racemic 2-octyl-DL-glycine ester using a chiral mandelic acid as a chiral resolving agent, and the product can be prepared by the following steps:
(1) a step of precipitating and filtering a salt of chiral 2-octylglycine ester having optical activity and chiral mandelic acid by subjecting a racemic 2-octyl-DL-glycine ester to optical resolution reaction with a chiral mandelic acid, which is a chiral resolving agent; and
   a step of separating and removing the chiral mandelic acid, which is a chiral resolving agent, from the salt of chiral 2-octylglycine ester and chiral mandelic acid obtained from the step (1) to produce a 2-octyl-L-glycine ester or a 2-octyl-D-glycine ester as a 2-octylglycine ester having optical activity.

The racemic 2-octyl-DL-glycine ester used as the raw material in the optical resolution reaction is an ester having an alkyl group having 1 to 4 carbon atoms, preferably a methyl ester. X, which is the substituent of the chiral mandelic acid of Formula 2 used as a chiral resolving agent, is hydrogen; a methoxy group; an alkyl group having 1 to 3 carbon atoms; fluorine; chlorine; bromine; or a cyano group, preferably hydrogen.

The optical resolution reaction may be performed in the presence of various organic solvents, and preferably includes at least one selected from the group consisting of ethyl acetate, isopropyl ether, methylbutyl ether, acetone and toluene.

Additionally, in the optical resolution reaction, the racemic 2-octyl-DL-glycine ester and chiral mandelic acid are mixed in an equivalent ratio of 1:0.5 to 1:1.5, preferably mixed in an equivalent ratio of 1:0.9 to 1:1.1.

If the equivalent ratio of the chiral mandelic acid of Formula 2 is less than 0.5, the optical resolution efficiency decreases, and if it exceeds 1.5, the optical resolution does not increase any more, making it uneconomical.

Various generally known methods may be used for the process of separating and removing the chiral mandelic acid from the salt of chiral 2-octylglycine ester and chiral mandelic acid obtained in the optical resolution reaction process, and preferably an extraction process may be used.

In the case of using an extraction process, when added to an organic solvent that does not mix with water and a basic aqueous solution, the chiral 2-octylglycine ester, which is fat-soluble, is extracted in the organic solvent, and the chiral mandelic acid, which is watersoluble, is dissolved in the aqueous solution, which allows easy separation and removal.

As the organic solvent, various organic solvents that do not mix with water may be used, and preferably methylene chloride or ethyl acetate is used.

The basic aqueous solution is not particularly limited in its type, but an aqueous solution comprising at least one selected from the group consisting of sodium hydroxide, sodium carbonate, and ammonia is used, and its pH is preferably 9 to 12.

The separated and removed 2-octyl-L-glycine ester or 2-octyl-D-glycine ester produced by the above method has high optical purity, and its optical purity can be analyzed using a chiral column. Specifically, it has an optical purity of 90/10 or more.

Meanwhile, when the obtained 2-octyl-L-glycine ester or 2-octyl-D-glycine ester is subjected to a hydrolysis process in the presence of the basic aqueous solution, which is a generally known method, the ester functional group is hydrolyzed and a 2-octyl-L-glycine or 2-octyl-D-glycine is obtained.

Additionally, when the salt of chiral 2-octylglycine ester and chiral mandelic acid is subjected to a hydrolysis process in the presence of the basic aqueous solution, the ester functional group is hydrolyzed to give a 2-octylglycine having optical activity.

Hereinafter, the present invention is described in further detail by way of examples. The following examples are only for illustrating the present invention, and the scope of the present invention is not limited to these examples.

### [Example 1] Production of 2-octyl-DL-glycine methyl ester

50.0 g of racemic 2-octyl-DL-glycine was added to 250 ml of methanol, and 58.00 g of thionyl chloride was added at 0°C, followed by stirring for 16 hours at 75°C and then slow cooling to room temperature. After the solution was concentrated, 300 ml of methylene chloride was added and stirred. Then, 250 ml of purified water, 14.98 g of potassium hydroxide, and 18.44 g of potassium carbonate were added, followed by stirring at room temperature for 20 minutes. Subsequently, the dichloromethane layer was concentrated to obtain 48.20 g of 2-octyl-DL-glycine methyl ester.

¹H-NMR (CDCl3, 400 MHz): δ 0.87 (t, 3H), 1.34 (m, 12H), 1.51 (m, 2H), 1.55 (m, 1H), 1.69 (m, 1H), 3.44 (dd, 1H), 3.71 (s, 3H).

### [Example 2] Production of salt of 2-octyl-L-glycine methyl ester and (S)-mandelic acid

10 g of the 2-octyl-DL-glycine methyl ester produced in Example 1 and 7.56 g of (S)-mandelic acid were added to 40 ml of ethyl acetate and 160 ml of isopropyl ether, followed by stirring for 2 hours at 70°C and then slow cooling to room temperature.

Subsequently, 6.55 g of salt of 2-octyl-L-glycine methyl ester and (S)-mandelic acid was obtained through precipitation and filtration (D/L ratio = 3/97).
Column: Chiralpak AD-H
Mobile phase: 10 % IPA, 90 % n-Hexane, 0.2 % ethanesulfonic acid
Detector: UV (210nm)
¹H-NMR (CD3OD, 400 MHz): δ 0.75 (t, 3H), 1.32 (m, 12H), 1.84 (m, 2H), 3.82 (s, 3H), 3.93 (t, 1H), 4.88 (s, 1H), 7.24 (m, 1H), 7.29 (m, 2H), 7.46 (m, 2H)

### [Example 3] Production of 2-octyl-L-glycine methyl ester

10.0 g of the salt of 2-octyl-L-glycine methyl ester and (S)-mandelic acid produced in Example 2 was added to 50 ml of methylene chloride, and then a solution where 1.13 g of sodium hydroxide and 1.50 g of sodium carbonate were dissolved in 50 ml of purified water was added, followed by fast stirring at room temperature for 20 minutes. Then, the organic layer was separated and concentrated to obtain 5.58 g of 2-octyl-L-glycine methyl ester (D/L ratio = 3/97).

¹H-NMR (CDCl3, 400 MHz): δ 0.88 (t, 3H), 1.33 (m, 12H), 1.51 (m, 2H), 1.54 (m, 1H), 1.70 (m, 1H), 3.42 (dd, 1H), 3.70 (s, 3H).

### [Example 4] Production of salt of 2-octyl-D-glycine methyl ester and (R)-mandelic acid

20 g of the 2-octyl-DL-glycine methyl ester produced in Example 1 and 14.8 g of (R)-mandelic acid were added to 90 ml of ethyl acetate and 350 ml of isopropyl ether, followed by stirring for 3 hours at 70°C and then slow cooling to room temperature.

Subsequently, 12.9 g of salt of 2-octyl-D-glycine methyl ester and (R)-mandelic acid was obtained through precipitation and filtration (D/L ratio = 96/4).

### [Example 5] Production of 2-octyl-D-glycine methyl ester

10.0 g of the salt of 2-octyl-D-glycine methyl ester and (R)-mandelic acid produced in Example 4 was added to 60 ml of methylene chloride, and then a solution where 1.15 g of sodium hydroxide and 1.60 g of sodium carbonate were dissolved in 60 ml of purified water was added, followed by fast stirring at room temperature for 30 minutes. Then, the organic layer was separated and concentrated to obtain 5.53 g of 2-octyl-D-glycine methyl ester (D/L ratio = 96/4).

### [Example 6] Production of 2-octyl-L-glycine

5.58 g of the 2-octyl-L-glycine methyl ester produced in Example 3 was added, 10 ml of methanol was added, and 1.70 g of sodium hydroxide was dissolved in 40 ml of purified water and added, followed by stirring at room temperature for 2 hours. The solution was neutralized to between pH 5.5 and 6.0 using 35% hydrochloric acid. The precipitated solid was filtered to obtain 4.65 g of 2-octyl-L-glycine (D/L = 0.5/99.5).

¹H-NMR (D₂O, 400 MHz): δ0.68 (s, 3H), 1.08 (m, 12H), 1.36 (m, 2H), 3.03 (m, 1H).

### [Example 7] Production of 2-octyl-D-glycine

The 2-octyl-D-glycine methyl ester produced in Example 5 was treated in the same manner as in Example 6 to obtain 2-octyl-D-glycine (D/L = 99.4/0.6).

¹H-NMR (D₂O, 400 MHz): δ0.67 (s, 3H), 1.07 (m, 12H), 1.36 (m, 2H), 3.02 (m, 1H).

## Claims

1. A method for producing a 2-octylglycine ester having optical activity, the 2-octylglycine ester being represented by Formula 1, **characterized in that** a 2-octyl-DL-glycine ester is reacted with a chiral mandelic acid represented by Formula 2 as a chiral resolving agent: 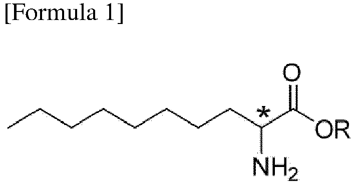
wherein R is an alkyl group having 1 to 4 carbon atoms, and
* is a chiral center; 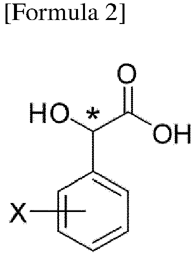
wherein X is hydrogen; a methoxy group; an alkyl group having 1 to 3 carbon atoms; fluorine; chlorine; bromine; or a cyano group, and
* is a chiral center.

2. The method for producing a 2-octylglycine ester having optical activity according to claim 1,
**characterized in that** R of Formula 1 is a methyl group and X of Formula 2 is hydrogen.

3. The method for producing a 2-octylglycine ester having optical activity according to claim 1,
**characterized in that** the equivalent ratio of the 2-octyl-DL-glycine ester and the chiral mandelic acid is from 1:0.9 to 1:1.1.

4. The method for producing a 2-octylglycine ester having optical activity according to claim 1,
**characterized in that** the method comprises:
(1) a step of precipitating and filtering a salt of chiral 2-octylglycine ester and chiral mandelic acid obtained from optical resolution reaction of the 2-octyl-DL-glycine ester with the chiral mandelic acid, which is a chiral resolving agent; and
(2) a step of separating and removing the chiral mandelic acid, which is a chiral resolving agent, from the salt of chiral 2-octylglycine ester and chiral mandelic acid obtained from the step (1).

5. The salt of chiral 2-octylglycine ester and chiral mandelic acid obtained from the step (1) of claim 4.

6. A method for producing 2-octylglycine having optical activity, **characterized in that** the 2-octylglycine ester having optical activity produced according to the production method of claim 1 is subjected to hydrolysis in a basic aqueous solution.

7. A method for producing 2-octylglycine having optical activity, **characterized in that** the salt of chiral 2-octylglycine ester and chiral mandelic acid obtained from the step (1) of claim 4 is subjected to hydrolysis in a basic aqueous solution.
